(19) Europäisches Patentamt
European Patent Office
Office européen des brevets

(11) Veröffentlichungsnummer: **0 295 495 B1**

(12) # EUROPÄISCHE PATENTSCHRIFT

(45) Veröffentlichungstag der Patentschrift: **23.10.91**

(51) Int. Cl.5: **A61K 9/18**, A61K 31/47, A61K 31/44, A61K 31/535

(21) Anmeldenummer: **88108732.4**

(22) Anmeldetag: **01.06.88**

Die Akte enthält technische Angaben, die nach dem Eingang der Anmeldung eingereicht wurden und die nicht in dieser Patentschrift enthalten sind.

(54) **Ionenaustauscherharze beladen mit Chinoloncarbonsäurederivaten, ihre Herstellung und Verwendung.**

(30) Priorität: **13.06.87 DE 3719764**

(43) Veröffentlichungstag der Anmeldung:
**21.12.88 Patentblatt 88/51**

(45) Bekanntmachung des Hinweises auf die Patenterteilung:
**23.10.91 Patentblatt 91/43**

(84) Benannte Vertragsstaaten:
**AT BE CH DE ES FR GB GR IT LI NL SE**

(56) Entgegenhaltungen:
**EP-A- 0 210 513**
**DE-A- 3 542 972**

**CHEMICAL ABSTRACTS, Band 82, Nr. 18, 5. Mai 1975, Seite 284, Zusammenfassung Nr. 116048u, Columbus, Ohio, US; I. STIVIC et al.: "Ion exchangers and sustained release action of drugs", & ACTA PHARM. JUGOSL. 1974, 24(4), 249-58**

(73) Patentinhaber: **BAYER AG**

**W-5090 Leverkusen 1 Bayerwerk(DE)**

(72) Erfinder: **Lange, Peter Michael, Dr.**
**Walter-Flex-Strasse 9**
**W-5090 Leverkusen 1(DE)**
Erfinder: **Mitschker, Alfred, Dr.**
**Am Gartenfeld 50**
**W-5068 Odenthal(DE)**
Erfinder: **Naik, Arundev, Haribahai**
**Walter-Flex-Strasse 20**
**W-5090 Leverkusen 1(DE)**
Erfinder: **Rast, Hubert, Dr.**
**Hamberger Strasse 25**
**W-5090 Leverkusen 3(DE)**
Erfinder: **Scheer, Martin, Dr.**
**Herberts-Katernberg 7**
**W-5600 Wuppertal 1(DE)**
Erfinder: **Voege, Herbert, Dr.**
**Marin-Buber-Strasse 41**
**W-5090 Leverkusen 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

CHEMICAL ABSTRACTS, Band 105, Nr. 14, 6. Oktober 1986, Seite 376, Zusammenfassung Nr. 120819g, Columbus, Ohio, US; & JP-A-61 130 239 (DAIICHI SEIYAKU CO., LTD) 18-06-1986

Produktinformation zu Lewatit CNP (Bayer)

**Beschreibung**

Die vorliegende Erfindung betrifft Ionenaustauscherharze die mit Chinoloncarbonsäurederivaten beladen sind, Verfahren zu ihrer Herstellung und ihre Verwendung.

Es ist seit langem bekannt pharmazeutische Wirkstoffe an Ionenaustauscherharze zu binden um z.B. Wirkstoffe mit ausgeprägtem Eigengeruch besser anwendbar zu machen (CH-A- 383 552). Es ist auch bekannt pharmazeutische Wirkstoffe an Ionenaustauscherharze zu binden um eine gleichmäßige Freigabe des Wirkstoffs über einen längeren Zeitraum zu bewirken (EP-A-0 042 818).

Es ist ferner bekannt anthelmintische Wirkstoffe an Ionenaustauscherharze zu binden um den Geschmack der Wirkstoffe zu beeinflussen (DE-A-3 028 082).

Gegenstand der vorliegenden Erfindung sind:

Ionenaustauscherharze basierend auf schwach sauren kationischen Ionenaustauscherharzen die mit Chinoloncarbonsäurederivaten der Formel (I)

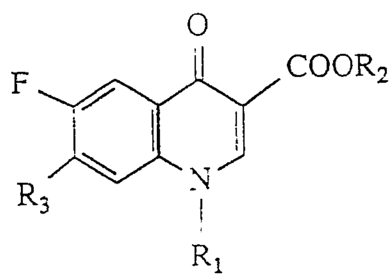 (I)

in welcher
$R^1$ für Cyclopropyl steht,
$R^2$ für Wasserstoff steht,
$R^3$ für

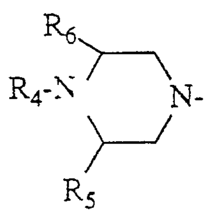

steht worin
$R^4$ fur Wasserstoff, Methyl, Ethyl steht,
$R^5$ für Wasserstoff steht,
$R^6$ für Wasserstoff steht,
beladen sind.

Verfahren zur Herstellung von Ionenaustauscherharzen basierend auf schwach sauren kationischen Ionenaustauscherharze, die mit Chinoloncarbonsäurederivaten der Formel (I)

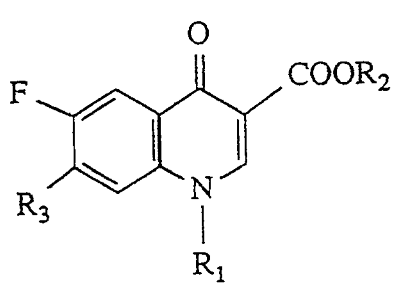 (I)

in welcher
$R^1$ für Cyclopropyl steht,
$R^2$ für Wasserstoff steht,

$R^3$ für

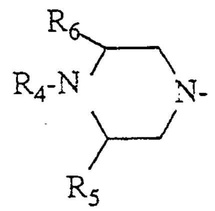

steht, worin
$R^4$ für Wasserstoff, Methyl, Ethyl steht,
$R^5$ für Wasserstoff steht,
$R^6$ für Wasserstoff steht,
beladen sind,
indem man schwach saure kationische Ionenaustauscherharze mit Lösungen oder Suspensionen von Chinoloncarbonsäurederivaten der Formel (I) in Wasser oder in polaren Lösungsmitteln behandelt.

Verwendung von Ionenaustauscherharzen gemäß Anspruch 1 basierend auf schwach sauren kationischen Ionenaustauscherharzen, die mit Chinoloncarbonsäurederivaten der Formel (I) beladen sind zur Herstellung antibakterieller Arzneimittel.

Arzneimittel, einschließlich Fütterungsarzneimittel, enthaltend Ionenaustauscherharze gemäß Anspruch 1, die basieren auf schwach sauren kationischen Ionenaustauscherharzen, die mit Chinoloncarbonsäurederivaten der Formel (I) beladen sind.

Feste, oral applizierbare Arzneimittel sowie Futterstoffe, enthaltend Ionenaustauscherharze gemäß Anspruch 1, die basieren auf schwach sauren kationischen Ionenaustauschern, die mit Chinoloncarbonsäurederivaten der Formel (I) beladen sind.

Als Ionenaustauscherharze kommen schwach saure kationische Typen in Frage, wobei deren Matrix gelförmig bzw. makroporös sein kann. Als Basismonomere für die Ionenaustauscher kommen polymerisierbare Monomere in Betracht, welche durch entsprechende Funktionalisierung zu Kationenaustauscherharzen umgewandelt werden können. Als Monomere seien beispielsweise (Meth)-acrylsäurester, (Meth)-acrylnitril sowie Styrol-Abkömmlinge genannt. Als weitere Comonomere werden zur Herstellung der Basispolymere Polyvinylverbindungen wie beispielsweise Divinylbenzol, Ethylenglykoldimethacrylat oder Methylenbisacrylamid eingesetzt. Auch Kondensationsharze, die zu Kationen austauscher führen, wie beispielsweise die bei der Umsetzung von Phenol, Formaldehyd mit Polyaminen entstehenden Harze sind als Träger für die Chinoloncarbonsäurederivate der Formel (I) geeignet.

Die verwendbaren Ionenaustauscher sind nicht neu. Die Herstellung dieser Harze ist beispielsweise in Ullmanns Enzyklopädie der techn. Chemie, Bd. 13, S. 299-305, 4. Auflage beschrieben. Die bevorzugten makroporösen Harze können unterschiedliche Porenvolumina aufweisen. Der Vernetzungsgrad der geeigneten Ionenaustauscherharze sollte bevorsingt bis zu 20 % und besonders bevorzugt bis zu 12 % betragen. Die Kunstharze liegen in Korngrößen von 50 bis 1300 µm, bevorzugt von 100 bis 300 µm vor.

Besonders erwähnt sei die Verwendung gemahlener Ionenauslauscher. Die Mahlung kann dabei vor oder nach der Beladung mit den Chinoloncarbonsäuren der Formel (I) erfolgen.

Handelsübliche Ionenaustauscherharze die eingesetzt werden können sind Lewatit®, Amberlite®, Dowex®.

Die Chinoloncarbonsäuren der Formel (I) und ihre Herstellung sind bekannt (DE-A-3 033 157).

Bevorzugt sind als Wirkstoffe die folgenden Chinoloncarbonsäuren genannt:
1-Cyclopropyl-6-fluor-1,4-dihydro-4-oxo-7-(1-piperazinyl oder 4-methyl- oder 4-ethyl-1-piperazinyl)-chinolon-3-carbonsäure.

Ganz besonders bevorzugt seien genannt Ciprofloxazin und Enrofloxazin.

Der Beladungsgrad der Ionenaustauscherharze mit den Chinoloncarbonsäurederivaten liegt in Abhängigkeit vom Harztyp zwischen 10 und 150 Gew.-% des getrockneten Ionenaustauschers.

Abgabeversuche zeigen, daß in Flüssigkeiten mit pH-Werten von 1 bis 3 der Wirkstoff besonders gut freigesetzt wird.

Die Herstellung der Ionenaustauscherharze die mit Chinoloncarbonsäurederivaten der Formel (I) beladen sind erfolgt in Wasser oder polaren organischen Lösungsmitteln wie z.B. Alkoholen wie Methanol oder Ethanol, Ketonen wie Aceton oder Gemische derselben Besonders bevorzugt ist Wasser. Ionenaustauscher und Wirkstoff werden dabei z.B. solange (z.B. 5 bis 24 Stunden) in Wasser bei Zimmertemperatur oder erhöhter Temperatur gerührt, bis der Wirkstoff vollständig gebunden ist.

Wie bereits erwähnt lassen sich die mit Chinoloncarbonsäurederivaten der Formel (I) beladenen Ionenaustauscher zur Herstellung von Arzneimitteln verwenden. Als solche seien bevorzugt Arzneimittel für Tiere genannt.

Für Tiere geeignete Arzneimittelzubereitungen sind z.B. solche, bei denen die Geschmacksverbesserung bei der Aufnahme eine Rolle spielt oder bei denen eine verzögerte Wirkstoff-Freisetzung nach der Applikation angestrebt wird.

Dies sind z.B. feste Zubereitungen wie Pulver, Premixe oder Konzentrate, Granulate, Pellets, Tabletten, Boli, Kapseln; Suspensionen die z.B. oral oder kutan angewendet werden. Sie werden hergestellt, indem man das wirkstoffbeladene Harz in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien, Lichtschutzmittel suspendiert.

Durch Zusatz von Substanzen, die die Viskosität erhöhen, können diese Suspensionen auch als sogenannte "halbfeste" Zubereitungen wie z.B. Salben verabreicht werden. Insbesondere können Formulierungen dieser Art zur Behandlung von Eutererkrankungen (Mastitis) eingesetzt werden oder als orale Paste bei Katzen, Hunden und Pferden verwendet werden.

Zur Herstellung fester Zubereitungen wird das wirkstoffbeladene Harz mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Zur Herstellung von Suspensionen werden die wirkstoffbeladenen Harze in einem Trägermedium möglichst homogen verteilt, gegebenenfalls unter Zuhilfenahme von anderen Hilfsstoffen wie Netzmittel, Konservierungsmittel oder viskositätserhöhende Stoffe.

Als Trägerflüssigkeiten seien genannt alle homogenen Lösungsmittel und Lösungsmittelgemische insbesondere aber Wasser.

Als Netzmittel (Dispergiermittel) seien genannt:

1. anionaktive Tenside einschließlich Emulgatoren wie Na-Laurylsulfat, Fettalkoholethersulfate, Mono/Dialkylpolyglykoletherorthophosphorsäureester-Monoethanolaminsalz, Ligninsulfonate oder Dioctylsulfosuccinat,

2. kationaktive Tenside, einschließlich Emulgatoren, wie Cetyltrimethylammoniumchlorid,

3. ampholytische Tenside, einschließlich Emulgatoren, wie Di-Na-N-lauryl-β-iminodipropionat oder Lecithin

4. nicht ionogene Tenside, einschließlich Emulgatoren, wie polyoxethyliertes Rizinusöl, polyoxyethyliertes Sorbitan-Monooleat, Sorbitan Monostearat, Ethylalkohol, Glycerinmonostearat, Polyoxyethylenstearat, Alkylphenolpolyglykolether, Pluronic®.

Besonders bevorzugt sind die nichtionischen Tenside.

Weitere Hilfsstoffe sind z.B.:

Farbstoffe, d.h. alle zur Anwendung am Tier zugelassenen Farbstoffe, die gelöst oder suspendiert sein können.

Antioxidantien wie z.B. Sulfite oder Metabisulfite wie Kaliummetabisulfit, Ascorbinsäure, Butylhydroxytoluol, Butylhydroxyanisol, Tocopherol.

Verdickungsmittel bzw. viskositätserhöhende Stoffe wie z.B. anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate, Alginate, Gelatine, Polyvinylpyrrolidon, Polyethylenglykole, Wachse, Gummi arabicum und Xanthan-Gummi oder Gemische der aufgeführten Stoffe.

Die wirkstoffbeladenen Ionenaustauscherharze können als solche oder in Form von Prämixen oder Futterkonzentraten dem Futter zugesetzt werden.

Prämixe und Futterkonzentrate sind Mischungen des Wirkstoffes mit einem geeigneten Trägerstoff.

Zu den Trägerstoffen zählen die Einzelfuttermittel oder Gemische derselben, sowie die weiter oben

genannten inerten Trägerstoffe.

Sie können darüber hinaus weitere Hilfsmittel enthalten, wie z.B.- Substanzen, die die Fließfähigkeit und Mischbarkeit regulieren, wie z.B. Kieselsäuren, Bentonite, Ligninsulfonate. Darüber hinaus können Antioxydantien wie BHT oder Konservierungsmittel wie Sorbinsäure oder Calciumpropionat hinzugefügt sein. Außerdem können den Prämixen zur Staubbindung Flüssigkeiten wie Paraffinöle, Pflanzenöle, Propylenglykole zugemischt werden.

Die wirkstoffbeladenen Harze können in den Formulierungen allein oder in Mischung mit anderen Wirktoffen, Mineralsalzen, Spurenelementen, Vitaminen, Eiweißstoffen, Farbstoffen, Fetten oder Geschmacksstoffen vorliegen.

Andere Wirkstoffe können z.B. Penicilline, deren Salze und Derivate sein, wei z.B. das Procain-Salz des Penicillin-G, oder Derivate desselben wie Oxacillin oder Cloxacillin.

Die Verabreichung der wirkstoffbeladenen Ionenaustauscherharze erfolgt bevorzugt zusammen mit dem Futter.

Zum Futter zählen Einzelfuttermittel pflanzlicher Herkunft wie Heu, Rüben, Getreide, Getreidenebenprodukte, Einzelfuttermittel tierischer Herkunft wie Fleisch, Fette, Milchprodukte, Knochenmehl, Fischprodukte, ferner die Einzelfuttermittel wie Vitamine, Proteine, Aminosäuren, z.B. DL-Methionin, Salze wie Kalk und Kochsalz. Zum Futter zählen auch Ergänzungs-, Fertig- und Mischfuttermittel. Diese enthalten Einzelfuttermittel in einer Zusammensetzung die eine ausgewogene Ernährung hinsichtlich der Energie- und Proteinversorgung sowie der Versorgung mit Vitaminen, Mineralsalzen und Spurenelementen sicherstellen.

Die Konzentration der Ionenaustauscher im Futter beträgt normalerweise etwa 0,01 - 500 ppm, bevorzugt 10 - 200 ppm.

## Herstellungsbeispiele

### Beispiel 1 (Vergleichspräparat mit stark saurem Ionenaustauscher)

100 ml einer Suspension von 5 g Enrofloxacin in entmineralisiertem Wasser werden mit 35 ml Lewatit® S 100 $H^+$ Form bis zum Klarwerden der wäßrigen Phase gerührt. Anschließend wird dieser Vorgang wiederholt bis auch nach 24-stündigem Rühren keine klare wäßrige Phase zu erhalten ist. Nach der Abtrennung des Harzes wird durch Differenzwägung des getrockneten Harzes vor und nach Beladung die aufgenommene Menge an Enrofloxacin bestimmt. Bei diesem Versuch werden 7,7 g Enrofloxacin gebunden.

### Beispiel 2 (Vergleichspräparat mit stark saurem Ionenaustauscher)

2714 ml Lewatit® SPC 108 $H^+$-Form werden zusammen mit 5000 ml entmineralisiertem Wasser und 782 g Enrofloxacin über Nacht gerührt. Das Harz wird von der klaren wäßrigen Phase isoliert und zweimal mit je einem Bettvolumen Wasser gewaschen. Nach dem 48-stündigen Trocknen im Vakuumtrockenschrank bei 60 °C erhält man so 1496 g des erfindungsgemäßen Präparates.

### Beispiel 3 (erfindungsgemäß)

80 ml Lewatit® CNP $H^+$-Form werden zusammen mit 500 ml entmineralisiertem Wasser und 8 g Enrofloxacin bei 60 °C gerührt. Nach 3-stündigem Rühren ist die wäßrige Phase klar. Der Ionenaustauscher hat den gesamten Wirkstoff gebunden.

## Formulierungsbeispiele (nicht erfindungsgemäße Präparate mit stark saurem Ionenaustauscher)

4. Premix für Fütterungsarzneimittel

| | |
|---|---|
| Enrofloxacin-Ionenaustauscher gemäß Beispiel 2 (4,85 g entsprechen 2,5 g Enrofloxacin) | 4,85 kg |
| Weizenmehl | 95,15 kg |
| | 100,00 kg |

Herstellung:

Die Substanzen werden in einem Mischer homogen vermischt.

| | |
|---|---|
| 5. Enrofloxacin-Ionenaustauscher gemäß Beispiel 2 (9,7 g entsprechen 5,0 g Enrofloxacin) | 9,7 kg |
| pflanzliches Öl | 4,0 kg |
| Kalksteinmehl | 86,3 kg |
| | 100,0 kg |

Das Kalkmehl wird mit dem Pflanzenöl vorgemischt und darin das Wirkstoffharz homogen verteilt.

**6. Zusammensetzung einer oralen Paste (z.B. für Hunde und Katzen)**

| | |
|---|---|
| **Enrofloxacin-Ionenaustauscher gemäß Beispiel 2 (1,88 g entsprechen 1 g Enrofloxacin) Korngrößen-Mittelwert 0,1 mm** | **1,88 kg** |
| **Glycerin** | **10,00 g** |
| **Benzylalkohol** | **1,00 g** |
| **Aroma** | **0,20 g** |

Methylhydroxypropylcellulosegel 2 % ad 100 ml

Herstellung:

Ein 2 %iges Methylhydroxypropylcellulosegel wird in üblicher Weise hergestellt. Darin werden Benzylalkohol und Aroma gelöst und der erfindungsgemäße Wirkstoff suspendiert.

**7. Zusammensetzung für ein Granulat**

| | |
|---|---|
| 1. Enrofloxacin-Ionenaustauscher gemäß Beispiel 2 (18,8 g entsprechen 10,0 g Enrofloxacin) | 18,8 g |
| 2. Milchzucker | 50,0 g |
| 3. Maisstärke | 29,2 g |
| 4. Gelatine | 2,0 g |
| | 100,0 g |

Die Substanzen 1, 2 und 3 werden gemischt. Aus 4 wird eine Gelatinelösung mit 22,0 g Wasser hergestellt. Damit wird die Mischung geknetet. Der feste Teig wird über eine Raspel zerkleinert und getrocknet und anschließend auf die gewünschte Korngröße gesiebt.

**8. Mischung**

| | |
|---|---|
| Enrofloxacin-Ionenaustauscher gemäß Beispiel 2 | 12,5 kg |
| Kalksteinmehl | 86 kg |
| polyoxyethyliertes Rizinusöl | 1,5 kg |
| | 100 kg |

Das Kalksteinmehl wird mit dem polyoxyethylierten Rizinusöl vorgemischt und dann der Wirkstoff zugegeben und homogen verteilt.

Anwendungsbeispiele

A. Bestimmung der Wirkstoffkonzentration im Blutserum von Schweinen die wirkstoffbeladenen Ionenaustauscher mit dem Futter verabreicht erhielten.

Ferkel mit einem Durchschnittsgewicht von 14,8 kg erhielten zweimal täglich je 0,3 kg Ferkelaufzuchtfutter das mit der angegebenen Menge Enrofloxacin vermischt war. Jeweils 1 Stunde nach der Fütterung wurde Blut entnommen und der Wirkstoffgehalt im Serum bestimmt. Es wurden folgende Werte festgestellt:

| Wirkstoffgehalt ppm | Wirkstoffspiegel µg/ml 1 h nach 1. Fütterung | 1 h nach 2. Fütterung |
|---|---|---|
| 100 | 0,3 | 0,5 |
| 200 | 0,6 | 0,6 |
| 400 | 1,1 | 1,0 |

B. Bestimmung der Akzeptanz von medikiertem Futter durch Schweine

Ferkel mit einem Durchschnittsgewicht von 14,8 kg erhielten zweimal täglich 0,3 kg Ferkelaufzuchtfutter das mit reinem Wirkstoff Enrofloxacin sowie mit Ionenaustauscherharz, das mit Wirkstoff beladen war versetzt war. Nach den angegebenen Zeiten wurde das Restfutter im Trog bestimmt. Dabei wurden folgende Werte festgestellt:

| Wirkstoffgehalt ppm | Tierzahl | Restfutter in % nach 15 | 30 | 60 min |
|---|---|---|---|---|
| 0 | 9 | 5 | 0 | 0 |
| 400 (reiner Wirkstoff) | 12 | 80 | 70 | 70 |
| 400 (Wirkstoff an Ionenaustauscher gebunden) | 11 | 10 | 0 | 0 |

**Patentansprüche**

1. Ionenaustauscherharze basierend auf schwach sauren kationischen Ionenaustauscherharzen die mit Chinoloncarbonsäurederivaten der Formel (I)

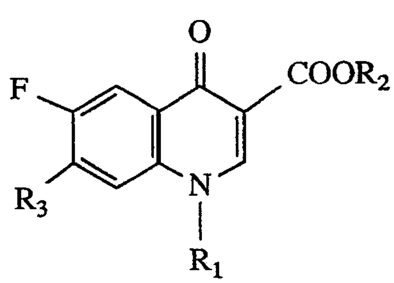

(I)

in welcher

$R^1$ für Cyclopropyl steht,

$R^2$ für Wasserstoff steht,

$R^3$ für

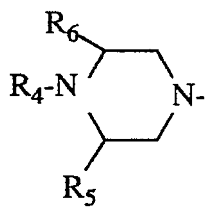

steht, worin

$R^4$ für Wasserstoff, Methyl, Ethyl steht,

$R^5$ für Wasserstoff steht,

$R^6$ für Wasserstoff steht,

beladen sind.

2. Verfahren zur Herstellung von Ionenaustauscherharzen basierend auf schwach sauren kationischen Ionenaustauscherharzen, die mit Chinoloncarbonsäurederivaten der Formel (I)

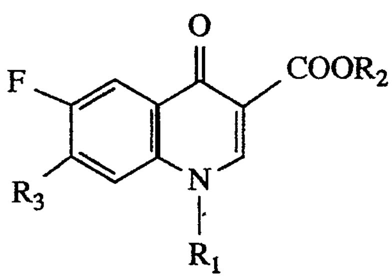

(I)

in welcher

$R^1$ für Cyclopropyl steht,

$R^2$ für Wasserstoff steht,

$R^3$ für

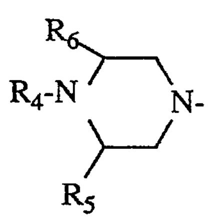

steht, worin

$R^4$ für Wasserstoff, Methyl, Ethyl steht,

$R^5$ für Wasserstoff steht,

$R^6$ für Wasserstoff steht,

beladen sind,

indem man schwach saure kationische Ionenaustauscherharze mit Lösungen oder Suspensionen von Chinoloncarbonsäurederivaten der Formel (I) in Wasser oder in polaren Lösungsmitteln behandelt.

**3.** Verwendung von Ionenaustauscherharzen gemäß Anspruch 1 basierend auf schwach sauren kationischen Ionenaustauscherharzen, die mit Chinoloncarbonsäurederivaten der Formel (I) beladen sind zur Herstellung antibakterieller Arzneimittel.

**4.** Arzneimittel, einschließlich Fütterungsarzneimittel, enthaltend Ionenaustauscherharze gemäß Anspruch 1, die basieren auf schwach sauren kationischen Ionenaustauscherharzen, die mit Chinoloncarbonsäurederivaten der Formel (I) beladen sind.

**5.** Feste, oral applizierbare Arzneimittel sowie Futterstoffe, enthaltend Ionenaustauscherharze gemäß Anspruch 1, die basieren auf schwach sauren kationischen Ionenaustauschern, die mit Chinoloncarbonsäurederivaten der Formel (I) beladen sind.

## Claims

**1.** Ion exchange resins based on weakly acidic cationic ion exchange resins which are loaded with quinolonecarboxylic acid derivatives of the formula (I)

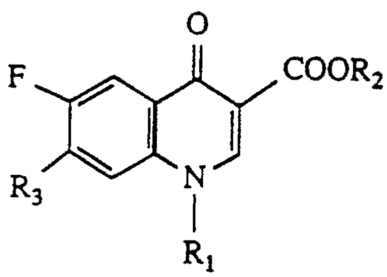

(I)

in which
$R^1$ represents cyclopropyl,
$R^2$ represents hydrogen,
$R^3$ represents

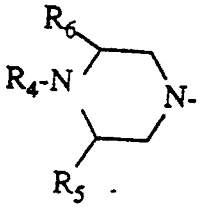

wherein
$R^4$ represents hydrogen, methyl or ethyl,
$R^5$ represents hydrogen and
$R^6$ represents hydrogen.

**2.** Process for the preparation of ion exchange resins based on weakly acidic cationic ion exchange resins which are loaded with quinolonecarboxylic acid derivatives of the formula (I)

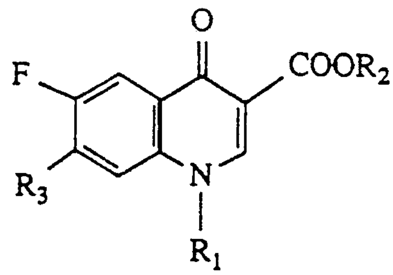

(I)

in which
$R^1$ represents cyclopropyl,
$R^2$ represents hydrogen,
$R^3$ represents

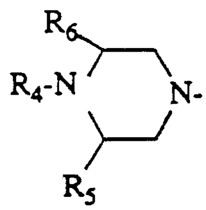

wherein
$R^4$ represents hydrogen, methyl or ethyl,
$R^5$ represents hydrogen and
$R^6$ represents hydrogen,
by treating weakly acidic cationic ion exchange resins with solutions or suspensions of quinolonecarboxylic acid derivatives of the formula (I) in water or in polar solvents.

3. Use of ion exchange resins according to Claim 1, which are based on weakly acidic cationic ion exchange resins which are loaded with quinolonecarboxylic acid derivatives of the formula (I), for the preparation of antibacterial medicaments.

4. Medicaments, including feed medicaments, which contain ion exchange resins according to Claim 1, which are based on weakly acidic cationic ion exchange resins which are loaded with quinolonecarboxylic acid derivatives of the formula (I).

5. Solid, orally administered medicaments and also feed-stuffs which contain ion exchange resins according to Claim 1, which are based on weakly acidic cationic ion exchangers which are loaded with quinolonecarboxylic acid derivatives of the formula (I).

**Revendications**

1. Résines échangeuses d'ions à base de résines échangeuses d'ions cationiques, faiblement acides, qui sont chargées avec des dérives d'acides quinolone-carboxyliques de formule (I)

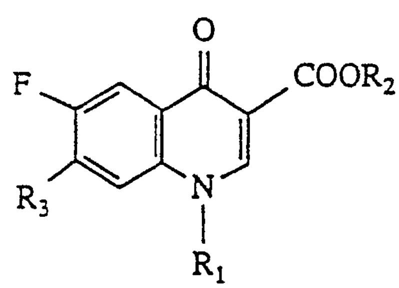

(I)

dans laquelle

$R^1$ représente un groupe cyclopropyle,

$R^2$ représente un atome d'hydrogène,

$R^3$ représente

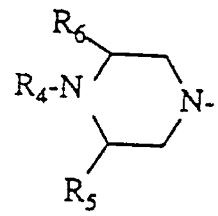

où

$R^4$ représente un atome d'hydrogène, un groupe méthyle, un groupe éthyle,

$R^5$ représente un atome d'hydrogène,

$R^6$ représente un atome d'hydrogène.

2. Procédé pour la préparation de résines échangeuses d'ions à base de résines échangeuses d'ions cationiques, faiblement acides, qui sont chargées avec des dérivés d'acides quinolone-carboxyliques de formule (I)

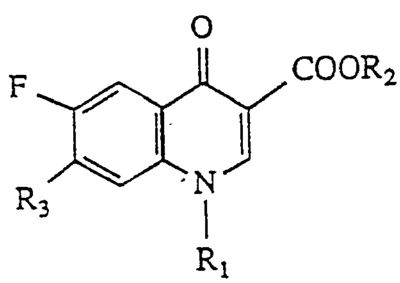

(I)

dans laquelle

$R^1$ représente un groupe cyclopropyle,

$R^2$ représente un atome d'hydrogène,

$R^3$ représente

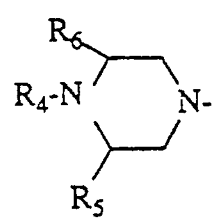

où

$R^4$ représente un atome d'hydrogène, un groupe méthyle, un groupe éthyle,

$R^5$ représente un atome d'hydrogène,

$R^6$ représente un atome d'hydrogène,

le procédé consistant à traiter des résines échangeuses d'ions cationiques, faiblement acides, avec des solutions ou des suspensions de dérivés d'acides quinolone-carboxyliques de formule (I) dans de l'eau ou dans des solvants polaires.

3. Utilisation de résines échangeuses d'ions selon la revendication 1, à base de résines échangeuses d'ions cationiques, faiblement acides, qui sont chargées avec des dérivés d'acides quinolone-carboxyliques de formule (I), pour la préparation de médicaments antibactériens.

4. Médicaments, y compris des médicaments pour affouragement, contenant des résines échangeuses d'ions selon la revendication 1, à base de résines échangeuses d'ions cationiques, faiblement acides, qui sont chargées avec des dérivés d'acides quinolone-carboxyliques de formule (I).

5. Médicaments solides applicables par voie orale, ainsi que des aliments pour animaux, contenant des résines échangeuses d'ions selon la revendication 1, à base de résines échangeuses d'ions cationiques, faiblement acides, qui sont chargées avec des dérivés d'acides quinolone-carboxyliques de formule (I).